(19) **Europäisches Patentamt / European Patent Office / Office européen des brevets**

(11) **EP 2 614 811 A1**

(12) **EUROPEAN PATENT APPLICATION**
published in accordance with Art. 153(4) EPC

(43) Date of publication:
**17.07.2013 Bulletin 2013/29**

(51) Int Cl.:
*A61K 8/06* (2006.01)     *B01F 11/02* (2006.01)
*B01J 19/10* (2006.01)     *A61Q 19/10* (2006.01)

(21) Application number: **11823837.7**

(22) Date of filing: **10.08.2011**

(86) International application number:
**PCT/RU2011/000603**

(87) International publication number:
**WO 2012/033433 (15.03.2012 Gazette 2012/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.09.2010 RU 2010137176**

(71) Applicant: **Getalov, Andrey Aleksandrovich Moscow 109548 (RU)**

(72) Inventor: **Getalov, Andrey Aleksandrovich Moscow 109548 (RU)**

(74) Representative: **Benatov, Emil Gabriel
Dr. Emil Benatov & Partners
Asen Peykov Str. No. 6
1113 Sofia (BG)**

(54) **METHOD FOR PRODUCING A COSMETIC EMULSION REMEDY**

(57)     The invention relates to medicine. The method for producing a cosmetic remedy comprises stagewise dispersion of the components at room temperature, wherein the components are introduced into an apparatus having a powerful hydroacoustic action, in which the components are dispersed and the emulsion is subjected to cavitation homogenization and then packaged. An acoustic cavitation mode is formed by a dual resonance effect within a continuous-flow mechanical oscillation system/channel of rectangular cross section in the final length, on the opposite sides of which channel sonic oscillations are generated in the parallel mode, with a standing wave being formed at the fundamental harmonic frequency for the specific channel wall, said sonic oscillations in turn forming a quasi-flat standing wave in a moving multi-phase medium, consisting of the ingredients being mixed, in the gap between the channel walls. The width of the channel gap h is selected by a multiple of one fourth of the wavelength being excited in the specific multi-phase medium by the channel walls. The oscillation amplitude of the channel wall is selected to be optimum for the various stages of preparing the emulsion and exceeds the acoustic cavitation threshold for the specific moving multi-phase medium being processed.

EP 2 614 811 A1

**Description**

The invention relates to the field of cosmetology and dermatology, and can be used in biology, pharmacology, cosmetics industry, veterinary and food industry, in particular in cosmetics industry - while development of technologies for obtaining cosmetic products for skin, hair and nails care.

It is known that the penetration of biologically active substances into the deeper tissues of the skin also depends on the dimensions and homogeneity of the oil phase of the cosmetic cream, which includes vegetable and essential oils, a number of important extracts and other fat-soluble ingredients. As a rule, in the technology of the cream tend to break up the oil phase droplets as small as possible.

In this case, along with liposomes of "oil in water" emulsion biologically active compounds can penetrate through skin tissues being dissolved in the oil phase of the emulsion and adsorbed at the structural interface.

There is a method of production of cosmetic cream being currently in use that includes the following stages of technological process:

- Weighing and melting of the raw materials;
- Preparation of fatty and aqueous phase;
- Emulsification;
- Cooling and perfume;
- Packing in the packing materials.

For the preparation of the aqueous phase the ingredients are heated to 75-80°C. To prepare the oil phase the ingredients are heated to 80-85°C. Further, the fat and aqueous phases are mixed. Under certain conditions (temperature, pH environment, the incorporation order) DNA and preservatives are added to a creamy mass (Russian Patent $N_o$ 2032399, IPC A 61 K7/00, A61K7/48).

The disadvantage of this method is the considerable power capacity of the cream production technologies and reducing of the biological activity of its components while the preparation of the product due to the fact that the production process of the emulsion is made by heating to 80 - 85°C, further there is homogenization of the two phases, which makes it more difficult the input of components and additives being critical to thermal decomposition (temperature up to 40 - 45°C) and at the same time being required for homogenization. These weak points significantly limit the application area of the method, in particular for the preparation of emulsions (creams, lotions, etc.) with the presence of natural vitamin supplements.

There is a method of production of cosmetic emulsion being currently in use comprising preparing basis by dispersing the vegetable oil, emulsifier and glycerol in the dispersion medium, followed by adding to the emulsion in the process of mixing of the biologically active substances of plant and animal origin. As the emulsifier polyethylene oxide gel is used, and dispersion is carried out at room temperature (Russian Patent $N_o$ 2126247, IPC 7 A 61 K 9/10, 7/48, publ. 2001).

The disadvantage of this analog method is the fact that for the intensification of the processes of dissolution and dispersion, as well as for preparation fine emulsions and suspensions the volumes are additionally equipped with high-speed stirring devices. Another disadvantage of this method is the inability of the homogenization process (cold emulsification), while the incorporation of biologically active substances into the cream to produce emulsions with submicron particle size.

The closest to the subject invention method is the method of manufacturing cosmetic products in the form of an emulsion comprising a dispersion of partially soluble components, emulsifiers, and biologically active substances (having any origin), solid powdered ingredients (sorbents or abrasives) in the solvent at room temperature where the components are added simultaneously or sequentially through individual dosing plants directly into the insonation camera of rotary-cavitation plant where cavitation emulsification process is implemented with simultaneous passing through the insonation chamber of emulsion complex "Mirra" (or any other) and an aqueous solution (Russian patent No. 2,240,782, IPC 7 A61K9/10, A61K9/50, A61K9/127, A61K7/48, 2004).

The nature of the subject invention simmers down to the use of cavitation homogenization (emulsification) concept to obtain high-efficient organic cosmetic and active cosmetic emulsion funds.

Certainly the application of rotary-cavitation treatment method for manufacturing cosmetic emulsions has several advantages over traditional methods of emulsification.

However, the process, or rather the mode of rotary-homogenizing into the insonation chamber of rotary machines requires more specification.

It is known [1] that in practice in the insonation chamber and in the channels of the stator of rotary rig different modes of cavitation of acoustic waves can be implemented.

Such wordings as "rotary cavitation plant with power hydroacoustic impact" and "mixture .... which is obtained in rotary cavitation plant by the cavitation emulsification method" should be specified. The question is only about the optimal selection of operating modes of rotary cavitation plants, as they depend on many factors.

For example, [/ 1 /, clause 2.2.2], technically the device implements cavitation mode in almost all the differential pressure, but it may occur as hydrodynamic cavitation mode [/ 1 /, clause 2.1.4] and the acoustic cavitation mode. And the peculiarities of resonance phenomena in rotary machines also include the existence of multiple resonances [/ 1 /, clause 1.1] and their complex interdependence with the design parameters of the plant, operation modes and the media characteristic. Each structural component is the generator of the particular frequency spectrum, which eventually superpose (superposition) to each other. In Section 4.4 the author [1] proved that the process of dissolution of sulfur in the oil mixture is optimum while implementation of the operation mode of rotary machine, exactly when it is excited by an acoustic pulse cavitation. There were devised a number of constructional decisions of rotor-stator pair and pipes nozzles for setting a resonant superposition of the frequencies for a particular technological process.

One of the authors of the prototype method in an interview [5] suggests that the frequency of exposure while rotary cavitation homogenization is 3 times the frequency of the current in the power supply, i.e., is about 150-180 Hz. According to the obtained results [/ 1 /, clause 2.2.4], this is probably one of the resonant modes, and there are resonances at higher frequencies with greater spectral density and a better Q factor, in particular, there are registered resonant frequencies ~ 540-580 Hz.

Thus, we can note the following significant disadvantages of the prototype:

- Difficult choices of the optimum operation mode of the rotary cavitation plant to obtain the required particle size and homogeneity of the final emulsion;
- The upper limit on the resonant frequency, which in practice will not exceed ~ 2000 Hz, due to the complex structure with many parts, each of which is the vibration transducer, which eventually superpose to each other and are not always in phase;
- In practice, it is known / 6 / that after passing through the homogenizer in the emulsion there is initiated a process of coalescence (interflowing) of droplets of the dispersed phase, even if the initial droplet size is small, and the is of dozens of nanometers and finally a stable emulsion is obtained, but with a wide range of size distribution of the dispersed phase size.

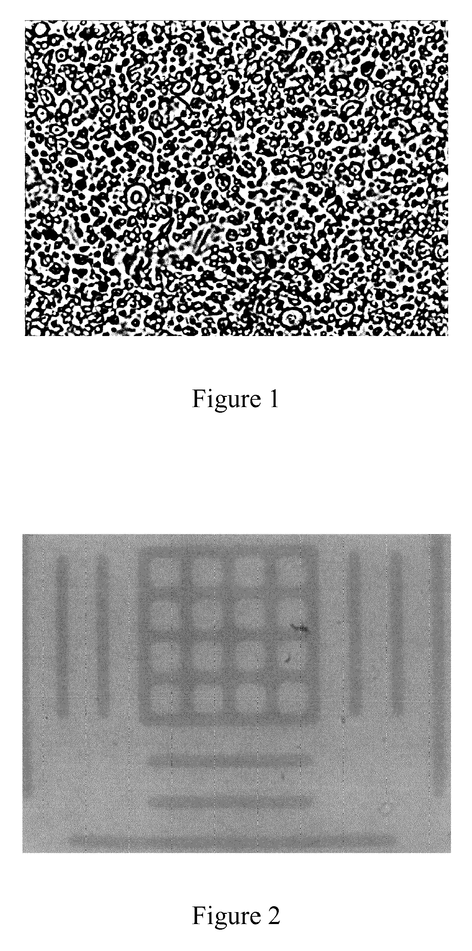

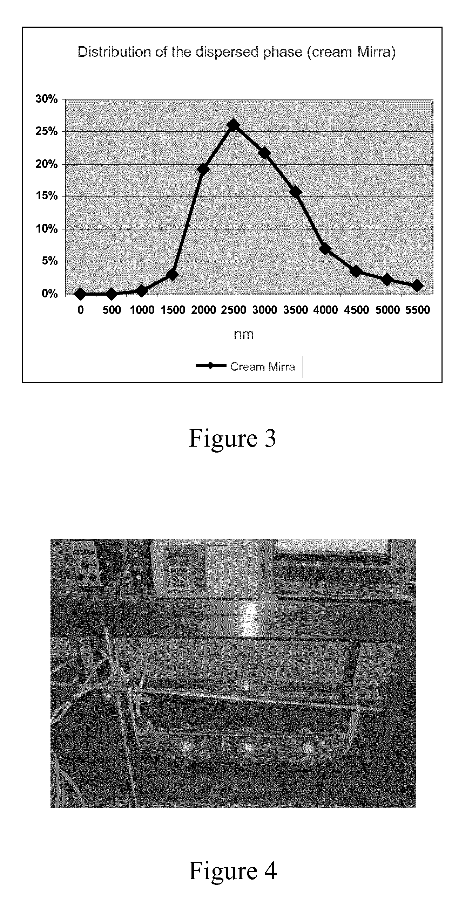

Figure 1 represents a photograph of cream produced by JSC Mirra-M (Nutritional cream with healing herbs) photograph after processing digital filter "smart sharpen" obtained with an optical microscope with an increase in 1000 and a digital packed with 5,2 megapixel resolution. To estimate the size of the dispersed phase object-micrometer FIG. 2 with a scale 10 mm was used. For digital image processing it was used the application Image Scope. The differential size distribution of the dispersed phase (processing results) are presented in Figure 3. As it can be seen from the obtained results used by Mirra company, rotor - cavitation homogenizers cannot provide long-term stable existence of the dispersed phase size of ~ 500-600 nm, which is considered close to the optimum for human skin, although immediately after completing of the homogenization process the share of this phase may be higher.

The research carried out by the company "DERMANIKA" of over 40 different creams (O/W and W/O emulsions) of leading Russian and foreign manufacturers have shown that the size distribution of the dispersed phase is close to each other for the products of 11 these manufacturers (the difference is not more than 2 times of the size of the fundamental mode) and is determined by the applied types of rotary homogenizers.

In addition the undertaken research took the opportunity to determine the optimal power and frequency characteristics of acoustic waves in the treated emulsion to produce significantly better performance characteristics on the size of the dispersed phase and the stability of the emulsion, to obtain correlations between the size of the dispersed phase and the frequency and level of homogeneity and Q-factor characteristic of oscillation channel system. There were found the effective methods of reducing peroxide value (specifies the number of free radicals) to the regulatory requirements.

The above research has been partially reported at the XIV International Research and Practice conference "Cosmetics and raw materials: safety and efficiency" in October 2009, where they were awarded second place and a diploma, there are publications in the specialized magazines / 7 /. There were implemented resonant modes being close to plane (quasi-plane) standing acoustic wave in the flowing emulsion in rectangular channel (the channel length is much more than the distance between the side walls).

In this case, in accordance with the criteria (the threshold) of cavitation [2, 3, 4] and the operation in resonant mode with maximum efficiency better characteristics for the intensification of combined physical-chemical, hydromechanical, heat-exchanging and mass-exchanging processes in the processing medium as well as the minimum size and homogeneity of fat (oil) phase obtained on output.

This technology is implemented on an industrial scale in the operating cosmetic production plant "CJSC EMANSI Laboratory." The photograph of the industrial plant is represented in Figure 4. The first products manufactured by this technology, hand cream Anti Smell Smoke (for smokers, against the influence of nicotine and smoke to hand skin), took the whole cycle of certification tests (Sanitary and epidemiological inspection report No. 77.01.12.915.P.006156.02.10 of February 03, 2010 and the Declaration of conformity, the results of which are also confirmed by independent testing of laboratory "Spectrum" (accreditation certificate No. ROSS RU.0001.21PSH50) with the corresponding protocol No. 19 dated December 22, 2009.

The aim of the invention is to reduce the average size of the dispersed phase wile obtaining any kind of emulsion (O/W or W/O) and improvement of the homogeneity (dimensional homogeneity).

This goal is achieved by the fact that the regime of quasi-plane resonant wave and acoustic cavitation is generated due to the double resonance effect in the flow mechanical oscillation rectangular channel system of finite length, on opposite sides of the channel the generating of sound waves with forming of a standing wave at the fundamental frequency for the given wall of the channel is in-phase provided, which in turn form a quasi-plane standing wave in a moving multi-phase consisting of mixed ingredients medium in the gap between the channel walls, at that the gap width of the channel is chosen multiple of quarter wavelength, excited in the multiphase medium by the channel walls:

$$\mathbf{h} = (k/4) * (\mathbf{C}/\mathbf{f}), \ k = 1, 2, 3,\ldots$$

where

  **f -** the frequency of fundamental harmonic of the standing wave of the channel wall, Hertz;
  **C -** the sound velocity in a multiphase medium, meter per second;
  **h -** the distance between the walls of the channel, meters;

and the amplitude of oscillation of the channel wall, is sorted out optimal for the different phases of manufacturing the emulsion is above the threshold of acoustic cavitation for the particular processed moving multiphase medium.

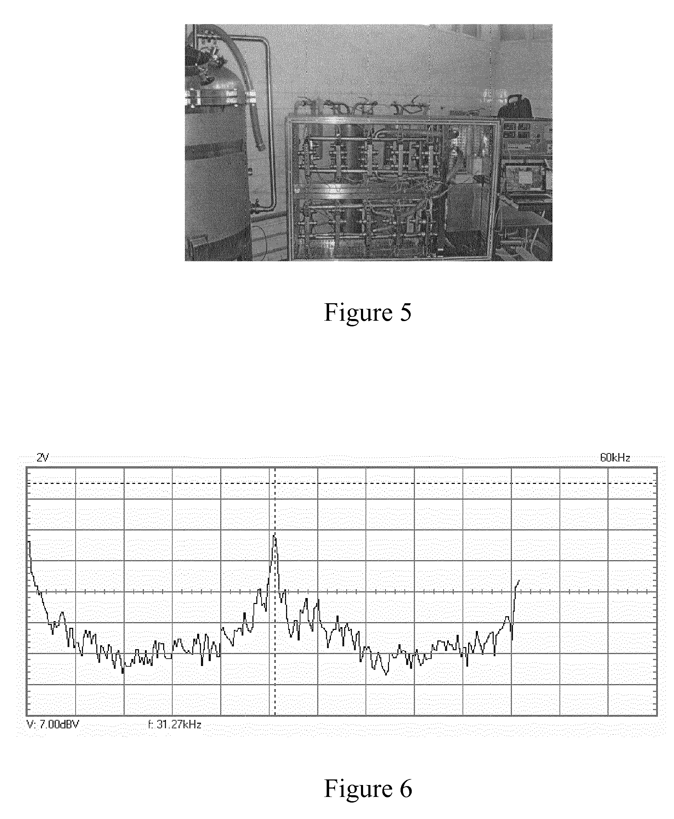

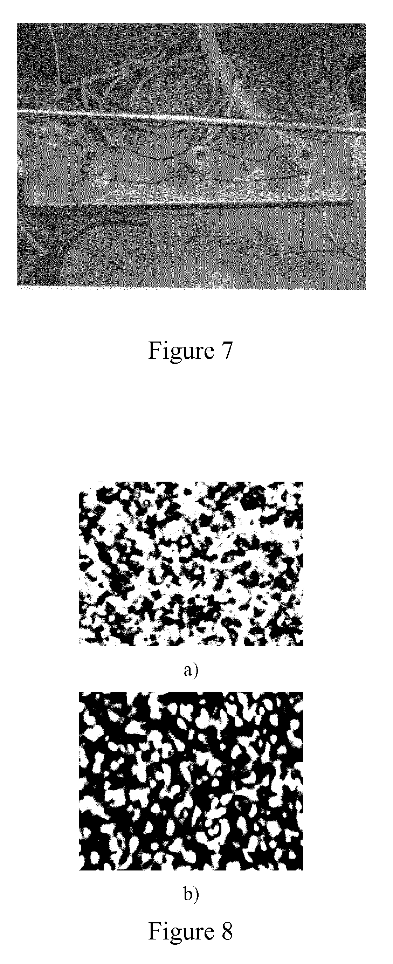

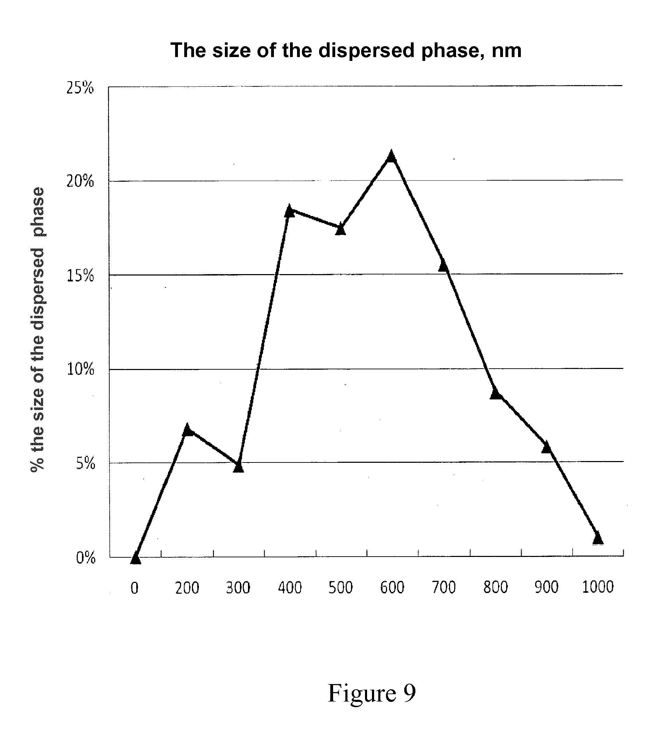

The character of the invention is explained by drawings: figure 1 Nutritional cream with healing herbs Mirra (processing by digital filter "smart sharpen"); figure 2 - Calibration scale, scale division 10 mm; figure 3 - The size distribution of the dispersed phase of the cream Mirra (prototype). Processing of the photograph shown in Figure 1.; figure 4 - The stage of laboratory testing; figure 5 - The appearance of an industrial plant; figure 6 - Typical spectrum of vibrations in the middle of the channel; figure 7 - Control of the nodal lines and loops on Chladni figures; figure 8 - Comparison of the structures of the creams in the same scale Cream Anti Smell Smoke (proposed technology); b) Cream Velvet Hands (of OAO "Kalina"); figure 9 - The size of distribution of the dispersed phase of the cream Anti Smell Smoke (proposed technology).

It is known / 8, 9 / that under certain conditions in any elastic plate of finite size standing waves occur, namely, there are such variations while which all points pulse with the same frequency and the same phase. The types of possible standing waves of the plate depend on its shape. In addition, the form of standing waves depends on the boundary conditions: the edges of the plate can be fixed or can be free. The frequencies of these self-oscillations (i.e., standing waves) depend not only on the size, shape of the plate, the boundary conditions, but also on the speed of wave propagation on the plate, and on a number of other characteristics. For a rectangular plate with fixed edges solution of the wave equation on a set of the frequencies of self-oscillation in the Cartesian coordinate system is given as / 9 /:

$$\omega = c\sqrt{k_x^2 + k_y^2} = c\sqrt{\left(j_x \frac{\pi}{L_x}\right)^2 + \left(j_y \frac{\pi}{L_y}\right)^2}$$

where c - speed of wave propagation on the plate;
$k_x$, $k_y$ -- wave numbers, the values of which are determined by the boundary conditions;
$L_x$, - length of the side of the plate directed along the axis Ox;
$L_y$ - - length of the side of the plate directed along the axis *Oy;*
$j_x$, $j_y$ - an integer equal to the number of crests of the wave along the respective sides of the plate.

For generating a quasi-plane wave in the gap between the channel walls is required to satisfy the conditions of the first fundamental mode when jx, jy are 1. The Fig. 4 represents one of the stages of laboratory tests to determine the self- frequencies of the channel, which further is supplied to complete the industrial unit Figure 5. For the measurements a measuring circuit is used, that passed a test in ROSTEST, the measuring circuit consists of piezo-acceleration transducer of 4344 type and the amplifier 2635 produced by the company Bruel & Kjaer, digital oscilloscope Velleman having function of the fast Fourier transforming with the registration of signals to a personal computer. A linear scale with a high resolution of 60 kHz is applied.

For initiating the ultrasonic vibrations a modified generator UZG 2-22 type with the auto-resonant frequency is used.

In Figure 6 there is a typical spectrum of vibrations of rectangular shape channel with the dimensions of 0.48 m and 0.1 m, the width of the gap 0,024 m. The calculated value of the fundamental mode to the above formula, is 29,7 kHz (with the velocity of longitudinal waves in a stainless steel ~ 5800 m/s), the experimentally observed value is 31,27 kHz. Checking of the self-frequency of the fundamental mode must be always carried out because in practice channels contain welded process connecting shafts for flushing and the theoretical calculation of the frequency channels is difficult / 8, 9 /.

In addition, an additional check is carried out by registering Chladni figures on the surface of the channel /Fig. 7/. The Q-factor of the channel is 4-6, which substantially improves the energy of emulsification process and eliminates the effect of other harmonics, which were previously observed.

The speed of sound in multi-phase media, such as cosmetic emulsions is not very different from the speed of sound value for water ((~ 1500 m/s), which allows, with sufficient accuracy for practical purposes, to calculate the optimum clearance **h** for the channel at a given resonance frequency. The wavelength is about 4,8 cm, respectively, for the superposition of waves within the channel; for a half-wave resonator the width should be ~3 2,4 cm.

The composition of cream Anti Smell Smoke is presented in the Table

| | | TOTAL QUANTITY, kg |
|---|---|---|
| **Name** | **Content %** | **Phase(temperature)** |
| DC 9045 | 0,70 | Active supplement 50 |
| DC 345 | 3,00 | Active supplement 50 |
| Lanette O | 2,20 | fat |
| Trylon B | 0,05 | water |
| Urea | 3,00 | water |
| Glycerin | 3,00 | water |
| Methylparaben | 0,30 | water |
| Propylparaben | 0,10 | water |
| Kathon CG | 0,05 | Active supplement 50 |
| Soybean oil Flavor | 0,30 | 50 degrees |
| Cetiol CC | 2,00 | fat |
| Skvalan | 0,50 | fat |
| Emulgade SE-PF | 1,20 | fat |
| edenor | 0,70 | fat |
| DC 200/100 | 1,00 | Active supplement 50 |
| Grindox 109 | 0,05 | Active supplement 50 |
| DC 200/100 | 1,00 | Active supplement 50 |
| Grindox 109 | 0,05 | Active supplement 50 |
| Biolin/P | 1,00 | Active supplement 50 |
| TegoSorb50 | 1,00 | fat |
| Mint gorophyt | 0,01 | fat |
| Crodamol ML | 0,70 | fat |
| Water | 79,14 | |
| TOTAL | 100,00 | |
| | | |
| Fat | 8,41 | |
| Watering | 6,35 | |
| Active+flavor | 6,10 | |
| water | 79,14 | |
| TOTAL | 100,00 | |

While preparation of the emulsion it has been determined three stages:

- Heating of oil phase and water with soluble components and their mixing (production of the base);
- The beginning of a homogenization and gradual cooling of the base;
- Adding of the active ingredients and fragrances at a temperature of about 45°C and homogenization at the constant temperature.

In stages 2 and 3, respectively, there were given different vibration amplitudes; cavitation control mode was provided by using temperature measurement at the inlet and outlet of the flow channel and according to the indicator of the power supplied to the piezo-oscillators.

In Figure 8 there is a photo showing in the same scale a comparison of cream Anti Smell Smoke and cream Velvet hands produced by OAO "Kalina".

In case of the size of the dispersed phase in the range of 400-700 nanometers the level of homogeneity amounts up 50-60 % /Figure 9/, that proves the achieving of the required result goal.

This structure of cosmetic emulsions for consumers has several new characteristics:

- the tactile and organoleptic characteristics (easy application and grinding, quick absorbency, the absence of a greasy film after application) are significantly improved. There were conducted market research (panels), where in the result of the anonymous survey 87 % of women and girls (65 respondents) selected the cream manufactured by the proposed method in comparison with the cream of the same composition, but prepared by traditional technology as the "best" according to organoleptic;
- to obtain a stable emulsion it is sufficient to use only 30-40 % of an emulsifier of the amount required for the preparation by traditional technology / 7 /. It is known that reduction of the emulsifier reduces the risk of destruction of the lipid barrier of the skin, i.e. contributes to maintaining healthy skin;
- The obtained emulsion has a pronounced rapid action. With continued use in as little as 5-7 days skin dryness disappeared, even when washing dishes by usual cleaning products, as it is indicated by the respondents.

Experiments show that the proposed method of manufacturing cosmetic emulsion allows to:

- obtain a smaller size of the dispersed oil (fat) phase;
- obtain a high level of homogeneity of the emulsion.

REFERENCES

1. V.M. Chervyakov, V.G. Odnolko "Use of hydrodynamic and cavitation phenomena in rotary machines". - Moscow: Publishing House of Mechanical Engineering, 2008.

2. Sirotyuk M. G. "Experimental research of ultrasonic cavitation. In Powerful ultrasonic fields", ed. L.D. Rosenberg, 1968.

3. V.A. Krasilnikov "Sonic and ultrasonic waves in air, water and solids"-M. Fizmatgiz, 1960.

4. L. Bergman, "Ultrasound and its application in science and tecnology".-M. : Foreign Literature, 1956.

5. A journal "Mirra Lux" The topic "Conversation of specialist. Interview with the Chief production technologist ", June, 1999.

6. Emulsions and emulsion technology, ed. K.J. Lissant, pt 1-2, New York, 1974.

7. V.L. Demenko, A.A. Getalov, T.V. Puchkova, E.A. Hoteenkova. "Effective method of reducing the content of the emulsifier in the production of cosmetic emulsions", the magazine "Raw materials and packaging" № 10 (101), page 12.

8. "Vibrations in technology". Reference in 6 volumes, ed. V. N. Chelomey, M., Mechanical Engineering, 1979.

9. Slobodyanyuk A.I. Physics textbook, Part 2, Paragraph 19, Publ. GENA, 2008.

**Claims**

1. A method of manufacturing of emulsion cosmetic products, including gradual dispersion of slightly-soluble components, such as vegetable oil, emulsifier and biologically active substances of different origin, solid powdered ingredients (sorbents or abrasives) in the solvent at room temperature at that the components are added to the plant with a powerful hydroacoustic effect, where dispersion of components and cavitation homogenization of emulsion with the further filling are made **characterized in that** the mode of acoustic cavitation is generated due to the double resonance effect in the flow mechanical oscillation rectangular channel system of finite length, on opposite sides of the channel the generating of sound waves with forming of a standing wave at the fundamental frequency for the given wall of the channel is in-phase provided, which in turn form a quasi-plane standing wave in a moving multiphase media consisting of mixed ingredients, at that the gap width of the channel is chosen multiple of quarter wavelength, excited in the multiphase medium by the channel walls:

$$\mathbf{h} = (\mathrm{k}/4)*(\mathbf{C/f}),\ \mathrm{k} = 1,\ 2,\ 3,\dots$$

where

> **f -** the frequency of fundamental harmonic of the standing wave of the channel wall, Hertz;
> **C -** the sound velocity in a multiphase medium, meter per second;
> **h -** the distance between the walls of the channel, meters;

and the amplitude of oscillation of the channel wall, is sorted out optimal for the different phases of manufacturing the emulsion is above the threshold of acoustic cavitation for the particular processed moving multiphase medium.

Figure 1

Figure 2

Figure 3

Figure 4

Figure 5

Figure 6

Figure 7

a)

b)

Figure 8

**The size of the dispersed phase, nm**

Figure 9

## INTERNATIONAL SEARCH REPORT

| International application No. |
|---|
| PCT/RU 2011/000603 |

**A.    CLASSIFICATION OF SUBJECT MATTER**

A61K 8/06 (2006.01) B01F 11/02 (2006.01)  B01J 19/10 (2006.01) Add.  A61Q 19/00 (2006.01)

According to International Patent Classification (IPC) or to both national classification and IPC

**B.    FIELDS SEARCHED**

Minimum documentation searched (classification system followed by classification symbols)

A61K 8/04-8/06, A61Q 19/00, B01F 11/02, B01J 19/10

Documentation searched other than minimum documentation to the extent that such documents are included in the fields searched

Electronic data base consulted during the international search (name of data base and, where practicable, search terms used)

PatSearch, Esp@cenet, CIPO, ChemIDplus Advanced, DEPATISnet, DWPI, EAPATIS, PAJ,

PCT Online, PubMed, RUPTO,USPTO, WIPO

**C.   DOCUMENTS CONSIDERED TO BE RELEVANT**

| Category* | Citation of document, with indication, where appropriate, of the relevant passages | Relevant to claim No. |
|---|---|---|
| A | RU 2240782 C1 (ZAO "MIRRA-M") 27.11.2004, p. 1 the claims | 1 |
| A | UA 59460 C2 (INSTITUT TEKHNICHESKOI TEPLOFIZIKI NATSIONALNOI AKADEMII NAUK UKRAINY) 15.09.2003, the abstract | 1 |
| A | US 2009/0166177 A1 (KIMBERLY-CLARK WORLDWIDE, INC.) 02.07.2009, the abstract | 1 |

☐   Further documents are listed in the continuation of Box C.          ☐          See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 30 November 2011 (30.11.2011) | 08 December 2011 (08.12.2011) |

| Name and mailing address of the ISA/ | Authorized officer |
|---|---|
| | |
| Facsimile No. | Telephone No. |

Form PCT/ISA/210 (second sheet) (July 1998)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- RU 2032399
- RU 2126247
- RU 2240782

### Non-patent literature cited in the description

- **V.M. CHERVYAKOV ; V.G. ODNOLKO.** Use of hydrodynamic and cavitation phenomena in rotary machines. Moscow: Publishing House of Mechanical Engineering, 2008
- Experimental research of ultrasonic cavitation. **SIROTYUK M. G.** Powerful ultrasonic fields. 1968
- **V.A. KRASILNIKOV.** Sonic and ultrasonic waves in air, water and solids. *M. Fizmatgiz,* 1960
- **L. BERGMAN.** Ultrasound and its application in science and tecnology. *M. : Foreign Literature,* 1956
- **A JOURNAL.** Mirra Lux. *The topic ''Conversation of specialist. Interview with the Chief production technologist,* June 1999
- Emulsions and emulsion technology. 1974
- **V.L. DEMENKO ; A.A. GETALOV ; T.V. PUCHKOVA ; E.A. HOTEENKOVA.** Effective method of reducing the content of the emulsifier in the production of cosmetic emulsions. *Raw materials and packaging,* vol. 101 (10), 12
- Vibrations in technology. M., Mechanical Engineering. 1979, vol. 6
- **SLOBODYANYUK A.I.** Physics textbook. Publ. GENA, 2008